# EUROPEAN PATENT SPECIFICATION

(11) **EP 1 340 457 B1**
(45) Date of publication and mention of the grant of the patent: **19.07.2006**
(21) Application number: 03076259.5
(22) Date of filing: 02.05.1996
(51) Int. Cl.: A61B 5/11, A01K 11/00

(54) **A method of automatically milking animals**
Verfahren zum automatischen Melken von Tieren
Méthode de traite automatique d'animaux

(30) Priority: 17.05.1995 NL 1000380
(43) Date of publication of application: 03.09.2003
(62) Divisional of application: 96201221.7
(73) Proprietor: MAASLAND N.V., 3147 PA Maassluis (NL)
(72) Inventor: van der Lely, Alexander, 3065 NA Rotterdam (NL); van der Lely, Olaf, 6300 Zug (CH); van der Berg, Karel, 2971 BR Bleskensgraaf (NL)
(74) Representative: Corten, Maurice Jean F.M.

(56) References cited:
- EP-A- 0 638 231
- WO-A-94/19931
- US-A- 4 247 758
- US-A- 4 618 861

## Description

The invention relates to a method of automatically milking animals according to the preamble of claim 1.

Such a method is known.

It is an object of the invention to provide an alternative method.

To this end the method comprises the features according to the characterizing portion of claim 1. In other words, the activity pattern determines the decision whether or not an animal presenting itself near the milking parlour will be milked

From US-4,247,758 and US-4,618,861 an animal identification and estrus system are known, per se, in which the number of animal movements are recorded and analysed.

For that purpose, in particular, it will have to appear from the activity pattern that the animal has already ruminated. Therefore, the above-mentioned method is furthermore characterized by the features of the characterizing portion of claim 2.

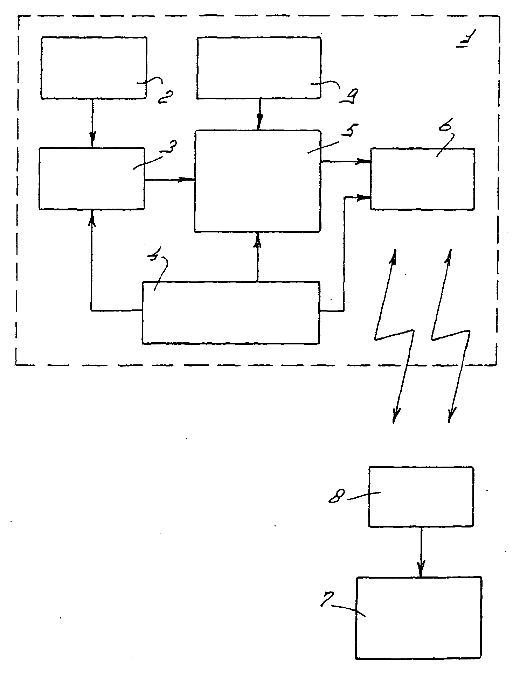

For a better understanding of the invention and to show how the same may be carried into effect, reference will now be made, by way of example, to the accompanying drawing, in which the animal activity meter according to the invention is shown schematically.

The animal activity meter 1 shown in the drawing, which meter can be worn by an animal around the neck or attached to one of the legs, is provided with a movement sensor 2, for the purpose of detecting the movements made by the animal. Said movement sensor 2 may be designed as a mercury switch. The number of movements established by means of the movement sensor 2 can be counted in a counting device 3, which is controlled by a control circuit 4 provided with a microprocessor. This control circuit ensures that the counting device 3 is reset in its starting position and will count thereafter, during a pre-fixed period of time, the number of movements made in said period of time which, optionally, can be adjusted, e.g. at five minutes, a quarter of an hour, half an hour, or at whatever value. After the adjusted period of time has elapsed, the count of the counting device is recorded in memory means 5, which are also controlled by the control circuit 4. Every time a count from the counting device 3 has been recorded in the memory means 5, the counting device 3 is reset in its starting position by the control circuit 4. In this manner, after some time, there is stored a series of counts in the memory means 5. Moreover, the animal activity meter 1 includes a transponder 6, by means of which the counts stored in the memory means 5 can be recorded in a computer 7, via a sensor 8 connected thereto. Although the sensor 8 may be arranged at a place chosen at random in a stable or cowshed, said sensor should preferably be disposed at the place where the animals are milked or near thereto, as this is the place where the animals go or are guided a number of times per twenty-four hours. As soon as the animals come in the vicinity of the sensor 8, the latter comes in contact with the transponder 6 in the animal activity meter 1, while the information present in the memory means 5 can be transmitted and recorded in the computer 7 via the sensor 8. For the purpose of supplying concentrate or for automatically milking, it is desirable that the animals can be identified at the place where they are fed or milked or near thereto. This identification is effected by means of an animal identification system provided with a transponder 6 including an electronic circuit, to be worn by the animal, in which system the animal number has been recorded, and a fixed sensor being in communication with a computer. It will be obvious that the animal activity meter and the animal identification system are adapted to be mutually integrated. For that purpose, in the embodiment shown, the memory means 5, besides the counts, also contain the animal number and, as soon as the animal comes in the vicinity of the sensor 8, these two data, via the transponder 6 and said sensor 8, are recorded in the computer 7. For each animal, there may be in the memory of the computer 7 a file, that is accessible with the aid of said animal number and in which the counts are stored. This file may additionally include all kinds of information relevant for feeding the animals or for automatically milking same.

When the animals are automatically milked while making use of a milking robot, the animals allowed to walk freely in an accommodation, such as a stable or cowshed or a meadow, can go individually, without human intervention, to the milking parlour in which the milking robot is disposed, where, after having been identified by means of the animal identification system, they may be milked. As there is usually supplied concentrate to the animals in the milking parlour, and the animals can go there voluntarily, it will be obvious that not every time the animals present themselves in the milking parlour they will actually be milked. It is possible to let the decision whether or not to milk an animal depend on the activity pattern as is established by means of the animal activity meter 1, as, for each animal, the activity pattern between two consecutive moments when the animal presents itself in the milking parlour or near thereto is established and recorded in the computer 7. When, every time the animal has presented itself in the milking parlour or near thereto, a series of counts is read out, there is obtained a permanently updated activity pattern. After having been milked, an animal will be in movement and consume fodder. After some time, the animal will lie down and ruminate. When, thereafter, the animal rises and begins to move, its movements will be less frequent than in the period prior to ruminating, as the lactation has sufficiently been resumed and the udder has become heavy, so that the freedom of movement of the animal is hampered. When the animal then goes again to the milking parlour, it will be milked. However, when the animal goes to the milking parlour and it appears from the activity pattern that this animal has not yet ruminated, then said animal has obviously presented itself too early and has to be removed from the milking parlour without having been milked. In other words, on the basis of the updated activity pattern, there can be decided whether or not to milk the animal when the latter presents itself in the milking parlour or near thereto. During the period of time the animal is lying down and is possibly ruminating, its movements are minimal. During that time, it is not strictly necessary that the counting device 3 is active and that the count is periodically recorded from said counting device in the memory means 5. The animal activity meter 1 may be provided with a specific sensor 9 for the purpose of measuring the distance to the ground and inciting a signal indicating that the animal is lying, standing or walking. The signals incited by this distance measuring sensor determine the moments when the animal lies down and rises, and can be recorded in the memory means 5. In other words, the periods of rest of the animal, obtained by means of the distance measuring sensor 9, can be stored directly in the memory means 5. These data can be recorded, in the same manner as the counts and the animal number, from the memory means 5, via the transponder 6 and the sensor 8, in the computer 7.

Besides directly from the updated activity pattern, the decision whether or not to milk an animal presenting itself in the milking parlour or near thereto can also be taken depending on the fact whether or not the sum of the counts, recorded in the computer 7 from the moment when the animal has been milked the previous time, exceeds a fixed value. After all, in the computer 7 there can be recorded how many movements a specific animal normally makes from the moment it has been milked until it has subsequently consumed fodder and has ruminated same, so that, as soon as an animal presents itself in the milking parlour or near thereto, the sum of the counts obtained since the previous milking run can be ascertained and the decision to milk the animal can be taken when this sum exceeds the threshold value for the number of movements to be made for this animal recorded in the computer.

The activity pattern detected for the individual animals can be averaged over a number of days. For example, by constantly making an average over the last week, there is obtained a possibly slowly varying value of a developing average. The animal activity as is read out from the memory means 5 every time the animal presents itself in the milking parlour or near thereto, can be compared with this developing average. In the case of values obtained that are significantly higher, the animal will be supposed to be on heat and in the case of values obtained being significantly lower, there will be an indication that the animal has one or more injured legs and/or is ill or at least has contracted a disease. Upon comparing the animal activity with the average value recorded in the memory, it has to be taken into consideration that these average values will not only differ per animal, but also depend on the fact whether the animal stays in a stable or cowshed or in a meadow. This means that for each animal there can be updated an average value for the period of time during which it stays in the stable or cowshed, as well as for the period of time during which it stays in the meadow.

## Claims

1. A method of automatically milking animals allowed to walk freely in an accommodation, such as a stable or cowshed or a meadow, and to go individually to a milking parlour including a milking robot where, after having been identified, they may be milked, wherein for individual animals, the activity pattern between two moments when an animal presents itself in the milking parlour or near thereto is established and recorded in a computer (7), **characterized in that**, on the basis of this activity pattern, there is decided in the computer (7) whether or not this animal will be milked.

2. A method as claimed in claim 1, **characterized in that**, when it appears from the activity pattern that an animal has had, after a period of movement, a period of rest, said animal, upon its presentation in the milking parlour or near thereto, will be milked.

## Patentansprüche

1. Verfahren zum automatischen Melken von Tieren, die in einer Unterkunft, wie z. B. einem Stall oder Kuhstall oder auf einer Wiese frei umherlaufen können und einzeln zum Melkstand gehen, in dem ein Melkroboter angeordnet ist, wo sie, nachdem sie mittels des Tieridentifikationssystems identifiziert worden sind, gemolken werden können, wobei für jedes Tier das Aktivitätsmuster zwischen zwei aufeinanderfolgenden Zeitpunkten, zu denen sich das Tier bei oder nahe dem Melkstand einfindet, erstellt und in einem Computer (7) aufgezeichnet wird,
**dadurch gekennzeichnet, daß** auf der Basis des Aktivitätsmusters in dem Computer (7) entschieden wird, ob das Tier gemolken wird oder nicht.

2. Verfahren nach Anspruch 1,
**dadurch gekennzeichnet, daß** das Tier, wenn aus dem Aktivitätsmuster hervorgeht, daß es nach einer Bewegungszeit eine Ruhezeit hatte, bei seinem Einfinden bei oder nahe dem Melkstand gemolken wird.

## Revendications

1. Procédé de traite automatique d'animaux autorisés à marcher librement dans un logement, tel qu'une écurie, une étable ou un pré, et à se déplacer individuellement jusqu'à une salle de traite comprenant un robot où, après avoir été identifiés, ils peuvent être traits, dans lequel, pour des animaux individuels, le modèle d'activité entre deux moments lorsqu'un animal se présente dans la salle de traite ou près de celle-ci est établi et enregistré dans un ordinateur (7), **caractérisé en ce que**, sur la base de ce modèle d'activité, il est décidé dans l'ordinateur (7) si cet animal doit être trait ou non.

2. Procédé selon la revendication 1, **caractérisé en ce que**, lorsqu'il semble, à partir du modèle d'activité, qu'un animal a eu, après une période de mouvement, une période de repos, ledit animal, lorsqu'il se présente dans la salle de traite ou près de celle-ci, sera trait.
